# EUROPEAN PATENT APPLICATION

(11) **EP 4 485 230 A1**
(43) Date of publication of application: **01.01.2025**
(21) Application number: 23774530.2
(22) Date of filing: 08.03.2023
(51) Int. Cl.: G06F 16/906, G16H 20/00

(54) **STORAGE AUXILIARY DEVICE, STORAGE AUXILIARY METHOD, AND PROGRAM**

(30) Priority: 25.03.2022 JP 2022049773
(71) Applicant: JVCKenwood Corporation, Yokohama-shi, Kanagawa 2210022 (JP)
(72) Inventor: SUGAHARA, Takayuki, Yokohama-shi, Kanagawa 221-0022 (JP)
(74) Representative: Wimmer, Hubert
(86) International application number: PCT/JP2023/008738
(87) International publication number: WO 2023/181931

(57) **Abstract**

A data acquiring unit (21) that acquires visual information or auditory information, and that acquires brain activation information; a tag generating unit (32) that generates a tag in which the visual information or the auditory information, the brain activation information, and a keyword are associated with an object identified from the visual information; and a storage unit (14) that stores the tag.

## Description

### Field

The present disclosure relates to a memory aid device, a memory aid method, and a computer program.

### Background

In recent years, technology for measuring brain activation information has developed, and the technology of brain-machine interface, which serve as an interface between the brain and an external device, has been becoming practical. As an example of a technique using such technology, there is one described in Patent Literature 1 below. An object search device described in Patent Literature 1 analyzes brain waves of a user and provides information about the object that the user is searching for, thereby assisting the user.

### Citation List

### Patent Literature

Patent Literature 1: JP-A2021-105935

### Summary

### Technical Problem

In recent years, dementia in old age is becoming a growing concern. When a memory impairment associated with dementia occurs, it becomes difficult to recall past events. Therefore, there is a need for development of a technique to assist people when they have difficulty in recalling past events.

The present disclosure has been achieved in view of the above problems, and aims to support recalling past memories.

### Solution to Problem

To solve the above problem and achieve the above object, a memory aid device according to the present disclosure comprising: a data acquiring unit that acquires any one of visual information and auditory information, and acquires brain activation information; a tag generating unit that generates a tag in which any one of the visual information and the auditory information, the brain activation information, and a keyword are associated with an object identified from the visual information; and a storage unit that stores the tag.

A memory aid method according to the present disclosure comprising: a step of acquiring any one of visual information and auditory information, and acquiring brain activation information; a step of generating a tag in which any one of the visual information and the auditory information, the brain activation information, and a keyword are associated with an object identified from the visual information; and a step of storing the tag.

A computer program according to the present disclosure that causes a computer to act as a memory aid device, the program causing the computer to execute: a step of acquiring any one of visual information and auditory information, and acquiring brain activation information; a step of generating a tag in which any one of the visual information and the auditory information, the brain activation information, and a keyword are associated with an object identified from the visual information; and a step of storing the tag.

### Advantageous Effects of Invention

According to the present disclosure, an effect of supporting recollection of past memories is produced.

### Brief Description of Drawings

FIG. 1 is a block diagram illustrating a memory aid device according to the present embodiment.
FIG. 2 is a flowchart illustrating a tag recording method in a memory aid device.
FIG. 3 is a flowchart illustrating a tag reproduction method in the memory aid device.

### Description of Embodiments

Hereinafter, a memory aid device, a memory aid method, and a computer program according to the present disclosure will be explained in detail with reference to the accompanying drawings. The embodiments described below are not intended to limit the present invention.

### Memory Aid Device

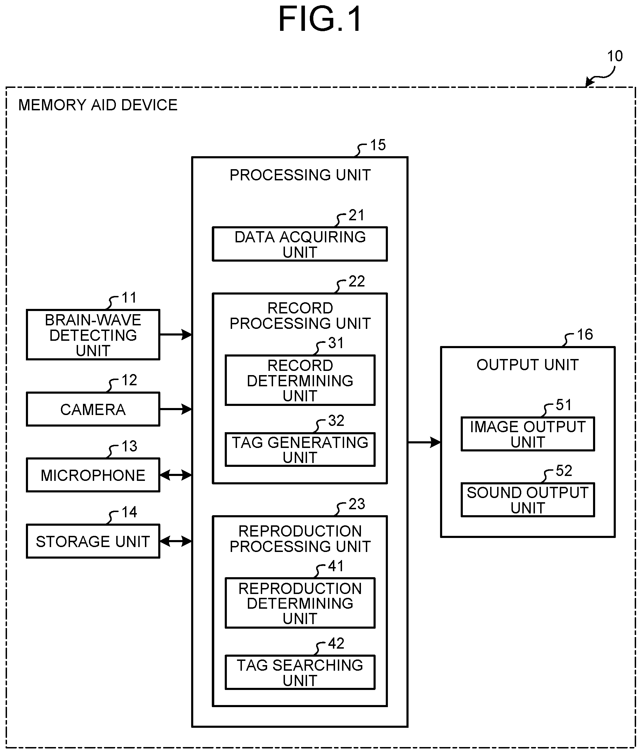

FIG. 1 is a block diagram illustrating a memory aid device according to the present embodiment.

As illustrated in FIG. 1, a memory aid device 10 is to support memory information of a user, and is, for example, mounted on the head of the user. The memory aid device 10 temporarily stores information of an object according to visual information and auditory information of the user. The memory aid device 10 supports recollection of the user by playing an image and a sound of the object when the user tries to recall the object.

The memory aid device 10 includes a brain-wave detecting unit 11, a camera 12, a microphone 13, a storage unit 14, a processing unit 15, and an output unit 16.

The brain-wave detecting unit 11 is connected to the processing unit 15. The brain-wave detecting unit 11 detects brain activation information of the user. Examples of the brain activation information include an oxygenated hemoglobin concentration, a deoxygenated hemoglobin concentration, and a total hemoglobin concentration in a cerebral blood flow of the user, and the like. For the brain-wave detecting unit 11, for example, a measurement device that performs measurement based on principles of a functional magnetic resonance imaging (fMRI), a functional near-infrared spectroscopy (fNIRS), a measurement device using an invasive electrode, a measurement device that performs measurement by a micromachine, placing the micromachine in a blood vessel in the brain, or the like can be used.

As for the brain activation information, for example, when the brain of the user is sectioned in a three-dimensional matrix constituted of voxels of several millimeters of smaller, a magnitude of activation of each voxel can be expressed as a brain activation map. The brain-wave detecting unit 11 is not limited to this configuration, and other kinds of devices may be used.

The camera 12 is connected to the processing unit 15. The camera 12 captures an image ahead of a line of sight of the user to record it. The image is preferable to be a moving image, but may be a still image. The camera 12 is worn by the user, but it may be mounted on an object (for example, glasses, a hat, a head-mounted display, or the like) worn by the user.

The microphone 13 is connected to the processing unit 15. The microphone 13 records sounds occurring ahead of a line of sight of the user. However, the microphone 13 may record sounds occurring around the user. The microphone 13 is worn by the user, but may be mounted on an object (for example, glasses, a hat, a head-mounted display, or the like) worn by the user.

The storage unit 14 is connected to the processing unit 15. The storage unit 14 stores processing performed by the processing unit 15. The storage unit 14 records a tag of the user generated by the processing unit 15 described later. The storage unit 14 may store the brain activation information detected by the brain-wave detecting unit 11, the image captured and recorded by the camera 12, and the sound recorded by the microphone 13. The storage unit 14 is constituted of a memory card, a solid state drive (SSD), an external storage device, and the like.

The processing unit 15 includes a data acquiring unit 21, a record processing unit 22, and a reproduction processing unit 23. The record processing unit 22 includes a record determining unit 31 and a tag generating unit 32. The reproduction processing unit 23 includes a reproduction determining unit 41 and a tag searching unit 42. Details of the processing unit 15 will be described later.

The processing unit 15 includes a processing device such as a central processing unit (CPU), and a storage device such as a random access memory (RAM) or a read only memory (ROM).

The output unit 16 outputs processing performed by the processing unit 15. The output unit 16 includes an image output unit 51 and a sound output unit 52. The image output unit 51 is, for example, a monitor, a head-mounted display, or the like. The sound output unit 52 is, for example, a speaker, a headphone, an earphone, and the like. However, the output unit 16 is not limited to such a configuration. For example, the output unit 16 may be configured to generate an electrical signal that evokes an image, a sound, and the like as the processing performed by the processing unit 15, and to provide electrical stimulation to the brain of the user by an electrode or the like.

### Processing Unit

The data acquiring unit 21 acquires visual information and auditory information of the user, and acquires the brain activation information. That is, the data acquiring unit 21 acquires the brain activation information (brain activation map) detected by the brain-wave detecting unit 11, the image captured and recorded by the camera 12, and the sound recorded by the microphone 13. The data acquiring unit 21 may store the acquired brain activation information of the user, image, and sound in the storage unit 14.

The record processing unit 22 identifies an object based on the brain activation information (the brain activation map), the image, and the sound acquired by the data acquiring unit 21 to generate a tag, and stores the generated tag in the storage unit 14.

First, the record determining unit 31 determines whether to generate a tag of the object based on the brain activation information (the brain activation map) of the user, the image, and the sound acquired by the data acquiring unit 21. The record determining unit 31 estimates, for example, a behavioral change of the user from the brain activation information (the brain activation map), and determines, when occurrence of a behavioral change is detected from the brain activation information (the brain activation map), that it is necessary to generate the tag at this moment.

The behavioral change of the user includes, for example, situations such as when the user starting conversations with someone, being approached by someone, receiving a phone call, having a visitor, going out, boarding a vehicle, shopping, and making payment.

When there is a behavioral change of the user, a activation state of the brain of the user changes. A brain activation map when there is no behavioral change of the user, and a brain activation map when there is a behavioral change are acquired in advance, and are stored in the storage unit 14. The brain activation map when there is no behavioral change of the user and the brain activation map when there is a behavioral change may be generated by a training model using machine learning. The record determining unit 31 determines that there has been a behavioral change of the user when a change amount between the brain activation map of the user acquired by the data acquiring unit 21 and the brain activation map when there is no behavioral change of the user stored in the storage unit 14 exceeds a determination value set in advance. Moreover, the record determining unit 31 determines that there has been a behavioral change of the user when a change amount between the brain activation map of the user acquired by the data acquiring unit 21 and the brain activation map when there is a behavioral change of the user stored in the storage unit 14 is equal to or smaller than the determination value set in advance.

Next, the tag generating unit 32 generates a tag in which the visual information, the auditory information, the brain activation information, and a keyword are associated with an object identified based on the visual information of the user. In this case, when the record determining unit 31 determines that there has been a behavioral change in the user, the tag generating unit 32 generates a tag. On the other hand, when the record determining unit 31 determines that there is no behavioral change in the user, the tag generating unit 32 does not generate a tag.

For example, when the user starts conversation with person A, the object is person A, and the keyword includes time and date of that time, a place (position), a color of clothes (for example, red or the like), a sex, or the like. The tag generating unit 32 generates a tag in which the object (person A), the keyword, the brain activation map, the image, and the sound are associated with one another.

Moreover, the tag generating unit 32 assigns priority based on the brain activation information (the brain activation map) when generating a tag. For example, while higher priority is assigned in a state in which the brain activation degree of a predetermined part of the brain of the user is low (a state in which the oxygenated hemoglobin concentration is low), lower priory is assigned in a state in which the brain activation degree of a predetermined part of the brain of the user is high (a state in which the oxygenated hemoglobin concentration is high). The priority may be assigned, for example, on a five-level scale based on the brain activity level in a predetermined part of the brain of the user.

The reproduction processing unit 23 searches for a tag of the object stored in the storage unit 14 when it is determined that there is a reproduction recollection signal of the object based on the brain activation information (the brain activation map) of the user acquired by the data acquiring unit 21.

First, the reproduction determining unit 41 determines whether the reproduction recollection signal of the object is present from the brain activation information (the brain activation map) of the user acquired by the data acquiring unit 21. The reproduction determining unit 41 determines that the user is engaged in recalling and memory support is necessary when it is determined that there is the reproduction recollection signal of the object based on the brain activation information.

When the user starts recollection to remember something, the activation state of the brain of the user changes, that is, it becomes an activated state. The brain activation map when the user is not recalling and the brain activation map when the user is recalling are acquired in advance, to be stored in the storage unit 14. The reproduction determining unit 41 determines that the user is engaged in recalling and that the reproduction recollection signal of an object is generated, for example, when a change amount between the brain activation information (the brain activation map) of the user acquired by the data acquiring unit 21 and the brain activation information (the brain activation map) when the user is not engaged in recalling stored in the storage unit 14 exceeds a pre-set determination value. The reproduction recollection signal is a signal generated based on the brain activation information as described above, and is a signal indicating a state in which the user is engaged in recalling. Moreover, the reproduction determining unit 41 determines that the user is engaged in recalling and the reproduction recollection signal of the object is present when a change amount between the brain activation map of the user acquired by the data acquiring unit 21 and the brain activation map when the user is engaged in recalling stored in the storage unit 14 is smaller than the pre-set determination value.

Next, the tag searching unit 42 searches for a tag of the object stored in the storage unit 14 based on the brain activation information. In this case, the tag searching unit 42 searches for the tag of the object when the reproduction determining unit 41 detects recalling of the user and determines that the reproduction recollection signal of the object is present. On the other hand, the tag searching unit 42 does not search for a tag when the reproduction determining unit 41 fails to detect recalling of the user and determines that the production recollection signal of the object is not present.

For example, the user is spoken to by person B, and tries to recall because the user cannot remember who person B is. At this time, the tag searching unit 42 detects a tag including the brain activation map stored in the storage unit 14 similar to the current brain activation map of the user, based on the brain activation information. Moreover, the tag searching unit 42 detects a tag including a keyword (red clothes) stored in the storage unit 14 using a color of clothes of person B currently viewed by the user as the keyword.

In this case, the tag searching unit 42 may acquire the keyword (red clothes) from the brain activation information of the user, or from the image of the camera 12.

Furthermore, the tag searching unit 42 changes the number of set keywords when detecting a tag based on the brain activation information. When a person is in a relaxed state with their eyes closed or is concentrating on a single task, alpha waves increase. On the other hand, when the brain is active, beta waves increase. When the tag searching unit searches for a tag of the object from the storage unit 14, the number of keywords for searching is set to a predetermined number. The tag searching unit 42 increases the number of keywords for searching to be more than the predetermined set number when the brain is in a tense state (beta waves) in which the brain of the user is active based on the brain activation information when searching for a tag of the object from the storage unit 14. On the other hand, the tag searching unit 42 decreases the number of keywords for searching to be less than the predetermined set umber when the brain of the user is in a relaxed state (alpha waves) based on the brain activation information, when searching a tag of the object from the storage unit 14.

### Memory Aid Method

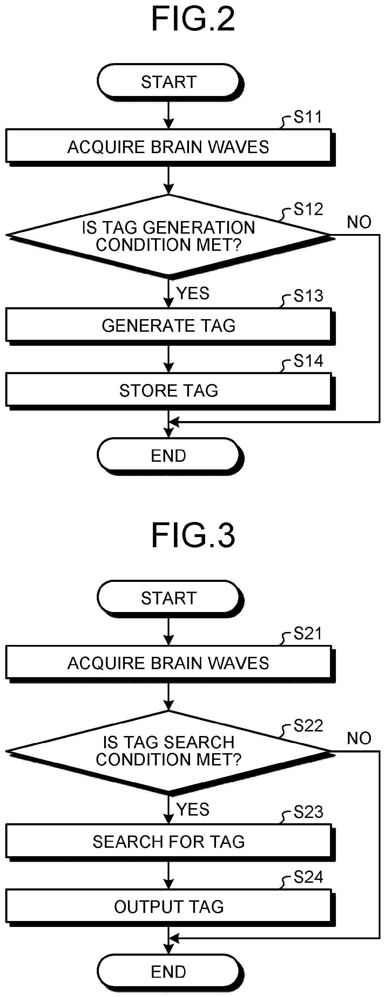

FIG. 2 is a flowchart illustrating a recording method in the memory aid device, and FIG. 3 is a flowchart illustrating a tag reproduction method in the memory aid device.

In the tag recording method, as illustrated in FIG. 1 and FIG. 2, the data acquiring unit 21 acquires brain waves of the user, that is, the brain activation information (the brain activation map) at step 511. At step S12, the record determining unit 31 determines whether a tag generation condition is met. The tag generation condition is detection of a behavioral change of the user. That is, the record determining unit 31 estimates behavioral changes of the user from the brain activation information (the brain activation map) of the user, and determines whether there has been a behavioral change in the user. When it is determined that the tag generation condition is not met, specifically, that there is not behavioral change in the user (NO), the record determining unit 31 exits from this routine.

On the other hand, when it is determined that the tag generation condition is met, that is, when it is determined that there has been a behavioral change in the user (YES), the record determining unit 31 generates a tag at step S13. That is, the tag generating unit 32 generates a tag in which the object, at least one keyword, the brain activation map, the image, and the sound are associated with one another. As for the image and sound, providing at least one is sufficient. At this time, the tag generating unit 32 may associate a priority based on the brain activation information (brain activation map) when generating a tag.

At step S14, the tag generating unit 32 stores the generated tag in the storage unit 14.

In the tag generation method, as illustrated in FIG. 1 and FIG. 3, the data acquiring unit 21 acquires brain waves of the user, that is, the brain activation information (the brain activation map) at step S21. At step S22, the reproduction determining unit 41 determines whether a tag search condition is met. The tag search condition is, for example, presence or absence of a reproduction recollection signal of the object. That is, the reproduction determining unit 41 determines, for example, whether the user is engaged in recalling by comparing the brain activation information (the brain activation map) with a brain activation map for determination stored in the storage unit 14. When it is determined that the tag reproduction condition is not met, that is, when it is determined that the user is not recalling (NO), the reproduction determining unit 41 exits this routine.

On the other hand, when the record determining unit 31 determines that the tag generation condition is met, that is, when it is determined that the user is engaged in recalling (YES), the tag searching unit 42 searched for a tag of the object stored in the storage unit 14 based on the brain activation information at step S33. The tag searching unit 42 detects, for example, a tag including a brain activation map stored in the storage unit 14 similar to the current brain activation map of the user. Moreover, the tag searching unit 42 detects a tag including the keyword. At this time, the tag searching unit 42 performs the search in descending order of priority included in tags when multiple tags are detected. Furthermore, the tag searching unit 42 may adjust the number of keywords used when searching the tag of the object, according to detection of alpha waves and beta waves based on the brain activation information.

At step S24, the tag searching unit 42 outputs the image and the sound associated with the tag searched based on the brain activation information to the output unit 16. The output unit 16 outputs the input image and the sound to let the user hear, thereby supporting recollection of the user. The user listens to the image or the sound output by the output unit 16, and can recall the object.

### Effects of Present Embodiment

The memory aid device according to the present embodiment includes the data acquiring unit 21 that acquires visual information or auditory information, and that acquires brain activation information, a tag generating unit 32 that generates a tag in which the visual information or the auditory information, the brain activation information, and a keyword are associated with an object identified from the visual information, and a storage unit 14 that stores the tag.

Therefore, by generating the tag in which the visual information or the auditory information, the brain activation information, and the keyword are associated with the object identified from the visual information of the user, to be stored, even if the user fails to memorize it, it is to be stored in the storage unit 14. Therefore, by providing a part of information stored in the storage unit 14 to the user as necessary, it is possible to support the user when recalling a past memory.

The memory aid device according to the embodiment includes the tag searching unit 42 that searches for a tag of an object stored in the storage unit 14 based on brain activation information, and an output unit 16 that outputs visual information or auditory information that are recorded in the tag. Therefore, when the user is trying to recall the object, it is possible to support the user to recall a past memory by searching the tag of the object based on the brain activation information, and by outputting the visual information or the auditory information recorded in the tag.

The memory aid device according to the present embodiment, the tag searching unit 42 changes the number of set keyword for detecting a tag based on brain activation information. Therefore, for example, when the user is in a state of tension, it is possible to provide accurate information to the user, and when the user is in a state of relaxation, the search criteria is broadened and the number of tags to be searched is increased, and it is thus possible to perform tag search according to the state of the user.

The memory aid device according to the present embodiment generates a tag associating a priority based on brain activation information. Therefore, for example, by prioritizing a state in which the brain activity of the brain of the user is low, visual information when the brain is not activated is to be searched in priority, and it is possible to prioritize and provide information that is difficult for the user to remember.

The memory aid device according to the present disclosure has so far been explained, but it may be implemented by various forms other than the embodiment described above.

The respective components of the memory aid device illustrated are of functional concept, and it is not necessarily required to be configured physically as illustrated. That is, specific forms of the respective devices are not limited to those illustrated, and all or some thereof can be configured to be distributed or integrated functionally or physically in arbitrary units according to processing loads, usage conditions, and the like of the respective devices.

The configuration of the memory aid device is implemented, for example, by a program loaded to a memory as software. In the embodiment described above, it has been explained as functional blocks implemented by cooperation of hardware or software. That is, these functional blocks can be implemented in various forms by only hardware, only software, or a combination of those.

The components described above include those easily thought of by a person skilled in the art, and those substantially the same. Furthermore, the configurations described above can be combined as appropriate. Moreover, various omissions, replacement, and changes of the configuration are possible within a range not departing from the gist of the present invention.

### Industrial Applicability

The preference determining device, the preference determining method, and the computer program according to the present disclosure can be used for a processing device, such as a computer, and the like.

### Reference Signs List

- 10: MEMORY AID DEVICE
- 11: BRAIN-WAVE DETECTING UNIT
- 12: CAMERA
- 13: MICROPHONE
- 14: STORAGE UNIT
- 15: PROCESSING UNIT
- 16: OUTPUT UNIT
- 21: DATA ACQUIRING UNIT
- 22: RECORD PROCESSING UNIT
- 23: REPRODUCTION PROCESSING UNIT
- 31: RECORD DETERMINING UNIT
- 32: TAG GENERATING UNIT
- 41: REPRODUCTION DETERMINING UNIT
- 42: TAG SEARCHING UNIT
- 51: IMAGE OUTPUT UNIT
- 52: SOUND OUTPUT UNIT

## Claims

1. A memory aid device comprising:
a data acquiring unit that acquires any one of visual information and auditory information, and acquires brain activation information;
a tag generating unit that generates a tag in which any one of the visual information and the auditory information, the brain activation information, and a keyword are associated with an object identified from the visual information; and
a storage unit that stores the tag.

2. The memory aid device according to claim 1, further comprising:
a tag searching unit that searches for the tag of the object stored in the storage unit based on the brain activation information; and
an output unit that outputs any one of the visual information and the auditory information recorded in the searched tag.

3. The memory aid device according to claim 2, wherein
the tag searching unit changes set number of the keyword used when detecting the tag based on the brain activation information.

4. The memory aid device according to any one of claims 1 to 3, wherein
the tag generating unit generates the tag associating a priority based on the brain activation information.

5. A memory aid method comprising:
a step of acquiring any one of visual information and auditory information, and acquiring brain activation information;
a step of generating a tag in which any one of the visual information and the auditory information, the brain activation information, and a keyword are associated with an object identified from the visual information; and
a step of storing the tag.

6. A computer program that causes a computer to act as a memory aid device, the program causing the computer to execute:
a step of acquiring any one of visual information and auditory information, and acquiring brain activation information;
a step of generating a tag in which any one of the visual information and the auditory information, the brain activation information, and a keyword are associated with an object identified from the visual information; and
a step of storing the tag.
